Europäisches Patentamt

European Patent Office

Office européen des brevets

(19)

(11) **EP 1 344 831 A1**

(12) **EUROPEAN PATENT APPLICATION**
published in accordance with Art. 158(3) EPC

(43) Date of publication:
**17.09.2003 Bulletin 2003/38**

(21) Application number: **01271868.0**

(22) Date of filing: **21.12.2001**

(51) Int Cl.[7]: **C12Q 1/02**, C12N 15/09,
C12N 5/10, G01N 33/15,
G01N 33/48, G01N 33/50,
G01N 33/566, G01N 33/68

(86) International application number:
**PCT/JP01/11301**

(87) International publication number:
**WO 02/052032 (04.07.2002 Gazette 2002/27)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **22.12.2000 JP 2000391234**

(71) Applicant: **CHUGAI SEIYAKU KABUSHIKI
KAISHA
Tokyo, 115-8543 (JP)**

(72) Inventors:
- **SUGO, Izumi
  Gotenba-shi, shizuoka 412-8513 (JP)**
- **YOSHIDA, Kenji
  Gotenba-shi, shizuoka 412-8513 (JP)**

(74) Representative: **Harding, Charles Thomas et al
D. Young & Co.
21 New Fetter Lane
London EC4A 1DA (GB)**

(54) **METHOD OF MEASURING CELL DEATH OF TARGET CELLS**

(57) A method, of determining cell death of target cells, comprising incubating target cells that contain a gene encoding a membrane-bound protein capable of inducing cell death and a gene encoding a marker protein, and determining the marker protein that leaks out of the cells due to cell death.

**EP 1 344 831 A1**

## Description

Field of the Invention

The present invention relates to a novel method of measuring the cell death of target cells.

Background Art

As methods of measuring cell death induced by reactions derived from membrane-bound proteins such as antibody-dependent cellular cytotoxicity (ADCC) activity, there is a method in which cells that incorporated $^{51}$Cr are used as the target cells and the amount of $^{51}$Cr leaked out of the cells as a result of cytotoxicity is determined. Generally, however, in methods that employ radioisotopes (RIs), experimental facilities are limited to within the RI control area and, from the viewpoint of controlling pollution and protecting laboratory personnel, strict controls are required in the implementation of experiments such as the limitation of amount of $^{51}$Cr annually used, the registration of laboratory personnel as RI workers, and the like.

As methods of detecting cell death that do not use RIs, an example in Japanese Unexamined Patent Publication (Kokai) No. 10-215868 determines β-galactosidase that leaked out of the cell due to cell death in order to detect cytotoxicity depending on the major histocompatibility complex (MHC) of cytotoxic T cells.

However, although the cytotoxicity (CTL activity) of cytotoxic T cells permits the determination of even a small amount of antigen bound by the MHC of target cells (Christinck E.R. et al., Nature 352 (1991), 67-70), ADCC activity depends on the amount of antigen expressed as the membrane-bound protein on target cells, and thus cannot be determined when the amount of antigen is small (Ohtomo T. et al., Biochem. Biophys. Res. Commun. 258(1999), 583-591). Thus, in order to determine cell death induced by reactions derived from membrane-bound proteins, it is necessary that target cells stably express the membrane-bound proteins.

Disclosure of the Invention

It is an object of the present invention to provide a method of determining cell death of target cells by detecting a marker that leaks out of the cells due to cell death, using target cells that express a membrane-bound protein in an amount sufficient to induce cell death by reactions derived from the membrane-bound protein, and express a non-RI marker as well.

After intensive and extensive research to resolve the above problems, the present inventors have successfully constructed transformed cells that are capable of expressing, in a stable manner, both a gene encoding a membrane-bound protein that can induce cell death and a gene encoding a marker protein, and thereby have completed the present invention.

Thus, the present invention provides a method of determining cell death of target cells, comprising incubating target cells that contain a gene encoding a membrane-bound protein capable of inducing cell death and a gene encoding a marker protein, and determining the marker protein that leaks out of the cells due to cell death.

The membrane-bound protein that can induce cell death may be any protein that induces cell death of cells due to the binding thereto of an antibody or a ligand, and includes, for example, the HM1.24 antigen (BST-2) protein or the FAS antigen protein. The above incubation may preferably be carried out in the presence of an antibody or a ligand against the membrane-bound protein capable of inducing the above cell death. The above incubation may preferably be carried out in the presence of effector cells.

The above target cells are preferably those that can provide a stable expression system of membrane-bound proteins. The above target cells are preferably CHO cells. The above marker is preferably an enzyme. The above marker is preferably β-galactosidase.

The present invention also provides a method of screening substances that either induce or inhibit cell death of target cells, comprising incubating target cells that contain a gene encoding a membrane-bound protein capable of inducing cell death and a gene encoding a marker protein together with test substances, and determining the marker protein that leaks out of the cells due to cell death.

The above membrane-bound protein capable of inducing cell death may be any protein that induces cell death of cells due to the binding of an antibody or a ligand, and includes, for example, the HM1.24 antigen (BST-2) protein or the FAS antigen protein. The above incubation may preferably be carried out in the presence of an antibody or a ligand against the membrane-bound protein capable of inducing the above cell death. The above incubation may preferably be carried out in the presence of effector cells.

The above target cells are preferably those that provide a stable expression system of membrane-bound proteins. The above target cells are preferably CHO cells. The above marker is preferably an enzyme. The above marker is preferably β-galactosidase.

The present invention also provides target cells for use in a method of determining cell death of target cells or screening substances that either induce or inhibit cell death of target cells, said cells containing a gene encoding a membrane-bound protein capable of inducing cell death and a gene encoding a marker protein, and leaking out of the cells due to cell death induced by reactions derived from said membrane-bound protein.

The above membrane-bound protein capable of inducing cell death may be any protein that induces cell death of cells due to the binding of an antibody or a ligand, and includes, for example, the HM1.24 antigen (BST-2) protein or the FAS antigen protein.

Preferably, the above marker protein leaks out by incubation in the presence of an antibody or a ligand against the above membrane-bound protein.

Preferably, the above marker protein leaks out by incubation in the presence of effector cells.

The above target cells are preferably those that provide a stable expression system of membrane-bound proteins. The above target cells are preferably CHO cells. The above marker is preferably an enzyme. The above marker is preferably β-galactosidase. Brief Explanation of the Drawings

Fig. 1 is a graph showing that the HM antigen is expressed in the cells that express β-galactosidase by the introduction of pβ-gal-IRES2-EGFP-F.

Fig. 2 is a graph showing β-galactosidase activity in the culture medium when the cells expressing β-galactosidase by the introduction of pβ-gal-IRES2-EGFP-F were lyzed according to the Galacton-star mammalian kit (the symbol <u>plus</u>) and when the cells were not lyzed (the symbol <u>minus</u>), indicating that β-galactosidase is expressed.

Fig. 3 is a graph showing that green fluorescent protein (EGFP-F) is expressed in the same cells as in Fig. 2. Comparison of Fig. 2 and 3 indicates that there is a rank correlation between the amount of β-galactosidase expressed and the amount of EGFP-F expressed.

Fig. 4 is a graph showing that when the cells expressing the membrane-bound protein HM into which pβ-gal-IRES2-EGFP-F has been introduced are incubated in the presence of anti-HM antibody and effector cells, cytotoxicity or the extracellular leakage of β-galactosidase takes place depending on the concentration of anti-HM antibody.

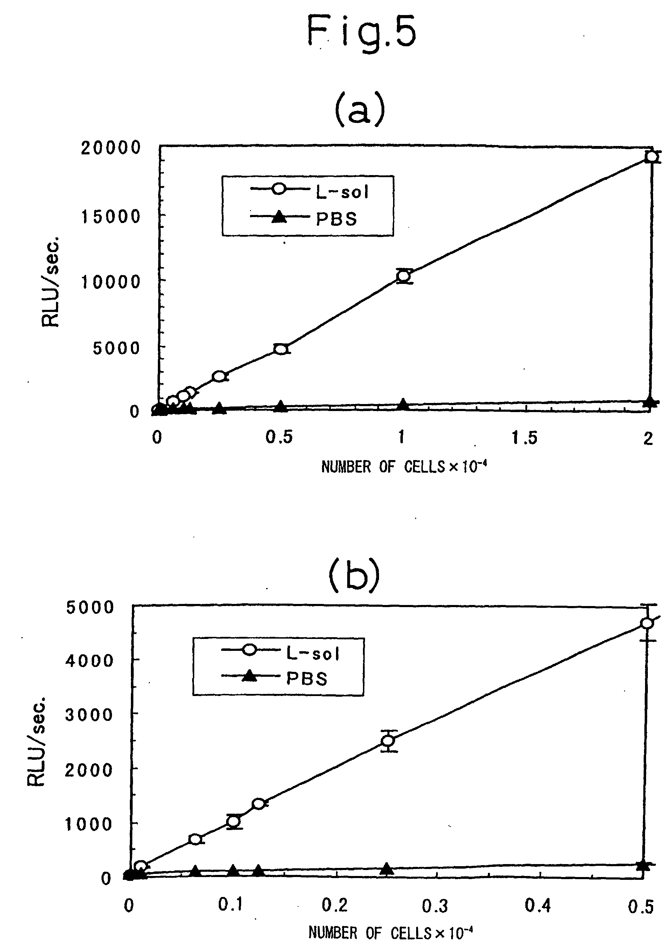

Fig. 5 is a graph showing that when the cells are lyzed with a surfactant in the experimental system of Fig. 4, the marker protein can be detected when there are not less than 100 cells, and no marker protein leaks out of the cells when the cells are not lyzed.

Fig. 6 is a graph showing that by stimulating commercially available PBMC with IL-2 in the experimental system of Fig. 4, there can be observed an antibody-dependent cytotoxicity and an antibody-non-dependent cytotoxicity. Best Mode for Carrying Out the Invention

The membrane-bound protein capable of inducing cell death may be any protein that induces cell death of cells due to the binding of an antibody or a ligand, and there can be mentioned the HM1.24 antigen (BST-2) protein, the FAS antigen protein, IAP, erbB2 and the like.

HM1.24 antigen protein is an antigen that is recognized by anti-HM1.24 antibody (Goto T. et al., Blood 84 (1994), 1922-1930) prepared by Kosaka et al. of Tokushima University. It has been elucidated that anti-HM1.24 antibody is a mouse monoclonal antibody obtained using a human myeloma cell line KPC-32 as antigen, recognizes HM1.24 antigen that is highly expressed on the surface of myeloma cells, induces ADCC activity in the presence of effector cells, and exhibits an anti-tumor effect (Ozaki S. et al., Blood 90 (1997), 3179-3186).

The HM1.24 antigen protein recognized by this antibody is a membrane-bound protein with a molecular weight of 29-33 kDa. As a result of gene cloning thereof, it was found to be identical with the BST-2 molecule (Ishikawa J. et al., Genomics 26 (1995), 527-534) that is highly expressed in stroma cells derived from patients with myeloma or rheumatism (Ohtomo T. et al., Biochem. Biophys. Res. Commun. 258 (1999), 583-591).

Fas antigen protein/CD95 is known as a protein that belongs to the TNF-receptor family present in the cell membrane, has the Death Domain in the cell (Yonehara S. et al., J. Exp. Med. 169 (1989) 1747-1756), and induces apoptosis through the Fas ligand (Cheng J. et al., Science 263 (1994) 1759) and anti-Fas antibody (Cifone M.G. et al., J. Exp. Med. 180 (1994) 1547) having an agonist effect.

The target cells of the present invention may be any cells that undergo cell death, for example apoptosis, and become dead, by a protein that can induce cell death, for example the above-mentioned HM1.24 antigen (BST-2) protein, the Fas antigen protein etc., and there can be used animal cells such as CHO cells, 293 cells, L cells, and NIH/3T3 cells.

The marker protein of the present invention may be any protein that can induce the above-mentioned cell death and, at the same time, can be expressed in the above target cells, leak out of the cells and can serve as a detectable marker per se, or can be labeled with a detectable marker. Such marker proteins include, for example, enzymes such as β-galactosidase, luciferase, alkaline phosphatase and peroxidase; luminescent substrates such as luciferin, CSPD (Tropix) and Galacton-star (Tropix).

β-galactosidase can be detected, for example, in the following manner. As a method of detecting and determining β-galactosidase with a high sensitivity, there is known a method that employs luminescent substrates. Thus, by allowing β-galactosidase to react at 1 μU (= about 1 pg = about $10^5$ molecules) - 100 mU with a luminescent substrate solution such as Galacton-star, the amount of luminescence corresponding to the amount of β-galactosidase can be obtained. The chemiluminescence can be measured using an optical instrument such as the ARVO-sx5 (Wallac).

In accordance with the present invention, in order to allow cell death to take place, when the membrane-bound protein that can induce cell death is the HM1.24 antigen (BST-2) protein, target cells that express this protein and a marker protein may be incubated in the presence of an antibody against said HM1.24 antigen (BST-2) protein and effector cells. Also, when the membrane-bound protein that can induce cell death is the Fas antigen protein, target cells that express this protein and a marker protein, at the same time, may be incubated in the presence of a ligand against said Fas antigen protein.

As effector cells, there can be used peripheral blood mononuclear cells (PBMCs), NK cells, cytokine-stimulated monocytes etc., and PBMCs, for example, may be prepared as follows.

When mononuclear cells are isolated from peripheral blood cells of normal healthy donors by density gradient centrifugation, an equal amount of PBS is added to the peripheral blood of normal healthy donors, onto which Ficoll-Paque PLUS (Pharmacia) is layered, and is then separated by centrifugation at 500 g for 30 minutes. After harvesting mononuclear cells and then washing them three times with RPMI1640 (GIBCO) containing 10% FBS (GIBCO), the cell number is adjusted with the same medium.

As antibody against proteins that can induce cell death, there can be used polyclonal antibody, monoclonal antibody, chimeric antibody, humanized antibody, antibody fragments and the like. These can be obtained or prepared by standard methods. Antibody against HM1.24 antigen, especially monoclonal antibody, chimeric antibody, humanized antibody etc., has already been described, and includes, for example, anti-HM1.24 antibody (Goto T. et al., Blood 84 (1994), 1922-1930), chimeric and humanized anti-HM1.24 antibody (WO98/14580) and the like, and the above anti-HM1.24 antibody can be obtained according to the method described in the above reference.

As ligands to the human Fas antigen protein, antihuman Fas monoclonal antibody CH-11 (Medical and Biological Laboratories Co., Ltd., No. SY-001) etc. is available.

The target cells of the present invention can be cultured according to, for example, a standard method. The cells that adhere to the surface of the culture vessel can be scraped according to a standard method such as trypsin treatment to obtain a cell suspension.

Cell death of the present invention can be determined by culturing the cells as above, and preparing the suspended cells in an α-MEM medium, for example, to a concentration of $10^3$ cells/ml or higher, to which the above antibody or ligand against the membrane-bound protein is added and incubated, and effector cells are further added to a concentration of 8/1-100/1 as an effector cell/target cell ratio and incubated. Alternatively, incubation can also be carried out in the presence of the above antibody or ligand and effector cells. The incubation time is 4 to 24 hours and the temperature is, for example, 37°C. The incubation causes cell death, with a result that the marker protein leaks out of the cells into the culture medium. Thus, the culture supernatant is harvested, and the marker protein therein may be determined as described above to measure the degree of cell death.

The determination of cell death of the present invention is particularly useful for the screening of ligands to the above membrane-bound protein (i.e., the determination whether or not a certain substance is a ligand) or the screening of agonists or antagonists against the membrane-bound protein (i.e., the determination whether or not a certain substance is an agonist or an antagonist). In the above method of measuring cell death in this screening, the incubation of the target cells with an antibody or a ligand thereto and/or effector cells may be carried out in the presence of test substances.

By comparing cell death or the amount of the leaked marker protein in a system (test system) in which the test substance was added and cell death or the amount of the leaked marker protein in a system (control) in which no test substance was added, it can be known whether or not the test substance is a ligand, or an agonist, or an antagonist.

Thus, when more marker protein leaked in the test system compared to the control system, the test substance is judged to be a ligand or an agonist, and when the result is the reverse, it is judged to be an antagonist.

Examples

The present invention will now be explained more specifically with reference to the following examples.

Example 1. Preparation of cells CHO#30 that express HM1.24 antigen (BST-2)

CHO #30 cells that express the HM1.24 antigen protein, a membrane-bound protein that induces cell death, were prepared in the following manner (Ohtomo T. et al., Biochem. Biophys. Res. Commun. 258 (1999), 583-591). Thus, an expression vector p3.19 (the above reference) encoding HM1.24 antigen was introduced into DHFR-deficient CHO cell line, which was subjected to selection with 500 µg/ml of G418, and then to the limiting dilution method to obtain CHO #30 cell line.

Example 2. Construction of an expression vector expressing the marker gene

A plasmid (pCMV/β-gal) (obtained from PE Biosystems, No. AV20C) comprising the β-galactosidase gene under the control of a cytomegalovirus promoter was digested with BamHI to obtain a DNA fragment containing the β-galactosidase gene. pIRES2-EGFP-F (Clontech) was digested and the above DNA fragment containing the β-galactosidase gene was integrated to construct a plasmid pβ-gal-IRES2-EGFP-F comprising a gene encoding β-galactosidase connected by IRES2 and green fluorescent protein (EGFP-F) under the control of a cytomegalovirus promoter. The plasmid further contains the neomycin resistant gene under the control of the SV promoter so as to permit drug selection of the transformed cells.

The plasmid was propagated using E. coli DH5α (Toyobo), and the plasmid collected was purified by the Qiagen Maxi column (Qiagen), the structure of which was confirmed by confirming that SacI digestion thereof gives an about 2.2 kb DNA fragment and by the confirmation of the base sequence of the junction site. As a result, pβ-gal-IRES2-EGFP-F was obtained.

Example 3. Introduction of pβ-gal-IRES2-EGFP-F into CHO #30 cells and cloning

(1) Transformation

After $1\times10^5$ cells of CHO #30 cells were plated into a 12-well plate and cultured overnight in a serum-containing α-MEM medium (containing nucleic acid: Gibco), and then, per 0.5 ml of serum-free OPTI-MEM (Gibco), (1) 3 μl of Lipofectamin/0.25 μg pIRES2-EGFP-F (as Moc), (2) 3 μl of Lipofectamin/0.25 g pβ-gal-IRES2-EGFP-F (D0.25/L3), (3) 3 μl of Lipofectamin/0.5 μg pβ-gal-IRES2-EGFP-F (D0.5/L3), or (4) 5 μl of Lipofectamin/0.25 μg pβ-gal-IRES2-EGFP-F (D0.25/L5) was added to the above cells that had been cleared of the medium and washed, followed by incubation for 4 hours, and then 1.5 ml of the serum-containing α-MEM (containing nucleic acid: Gibco) was further added and cultured for gene introduction.

(2) Confirmation of expression of the marker gene

In order to examine the expression of green fluorescent protein (GFP) as a marker for the introduced gene, the above cultured cells were scraped off the well with trypsin-EDTA, were then washed in phosphate buffered saline (PBS) and were suspended in PBS. Then, using EPICS-ELITE (BECKMAN COULTER), the cells that strongly fluoresce, by the expression of GFP, were harvested. The recovered cells were washed in α-MEM (nucleic acid added) medium containing 10% fetal calf serum (FCS), and cultured in α-MEM (nucleic acid added) medium containing 10% FCS or 0.5 mg/ml Geneticin (G418: Gibco) in a 6-well plate and stored.

As a result, when the transient expression of GFP was analyzed by flow cytometry on day two after gene introduction, about 40% of GFP-positive cells were observed for the CHO #30 cells into which pIRES2-EGFP-F had been introduced, and about 10% of GFP-positive cells were observed for the CHO #30 cells into which pβ-gal-IRES2-EGFP-F had been introduced, indicating that there is little difference due to the conditions (D0.25/L3, D0.05/L3, or D0.25/L5) at the time of gene introduction. The expression of GFP (as EGFP-F) was also observed by fluorescent microscope.

On the other hand, the amount of β-galactosidase expression was measured in the cells into which pβ-gal-IRES2-EGFP-F had been introduced. Thus, the cultured CHO #30 cells were scraped with typsin-EDTA, and were plated on a round-bottomed 96-well plate to a concentration of $1\times10^4$ cells/100 μl/well. To each well of the plate was added 100 μl of the cell lysis solution (Galacton-star assay kit) or 100 μl of the medium (10% FCS α-MEM), and then incubated for 4 hours in a $CO_2$ incubator. Twenty μl aliquots of the reaction mixture were taken into a 96-well plate, to which 100 μl of a reaction buffer containing Galacton-star was added. After reaction for 1 hour at room temperature, the amount of luminescence was measured by an ALVO-sx5.

As a result, the activity of β-galactosidase that spontaneously leaked into the culture medium during the above incubation was 4109.5 RLU (relative luminescence units) whereas the maximum leakage when incubated in the cell lysis solution was 25280.5 RUL.

However, when the expression of EGFP-F was analyzed on day six after gene introduction, about 1% of GFP-positive cells only remained in both of the MOC cells and pβ-gal-IRES2-EGFP-F-introduced cells. Thus, from the D0.5L3 cells that had been cultured for three weeks after gene introduction, GFP-positive cells were recovered by a cell sorter. As a result, about 300 GFP-positive cells were recovered from about 100,000 cells (recovery rate of about 0.3%), and when the recovered cells were grown for two weeks, the GFP-positive rate increased to about 30%.

(3) Monocloning of β-galactosidase-positive cells by limiting dilution

After the cell group recovered by the above cell sorter in which GFP-positive cells were concentrated was cultured in the α-MEM medium (nucleic acid added) containing 10% FCS and 0.5 mg/ml Geneticin, scraped with typsin-EDTA, and washed in PBS, the cell number was determined and then the cells were suspended in the α-MEM medium (nucleic acid added) containing 10% FCS and 0.5 mg/ml Geneticin to a concentration of one cell/ml. The cell suspension was dispensed in aliquots of 100 μl into each well of a 96-well plate, and the cells that grew in each well were divided in two groups, for activity measurement and for subculturing, were cultured in 90-well plates, and the culture for activity measurement was used to determine β-galactosidase activity.

β-galactosidase was determined by scraping the cultured cells from the well with typsin-EDTA, to which the medium was added to 100 μl, and then 100 μl of Galacton-screen was added and was allowed to react at room temperature for 1 hour followed by the measurement of amount of luminescence with ALVO-sx5. As a result, three lines of CHO #30-20, CHO #30-21, and CHO #30-40 were obtained as β-galactosidase high-expression lines.

(4) The amount of the HM antigen expressed of the β-galactosidase high-expression line

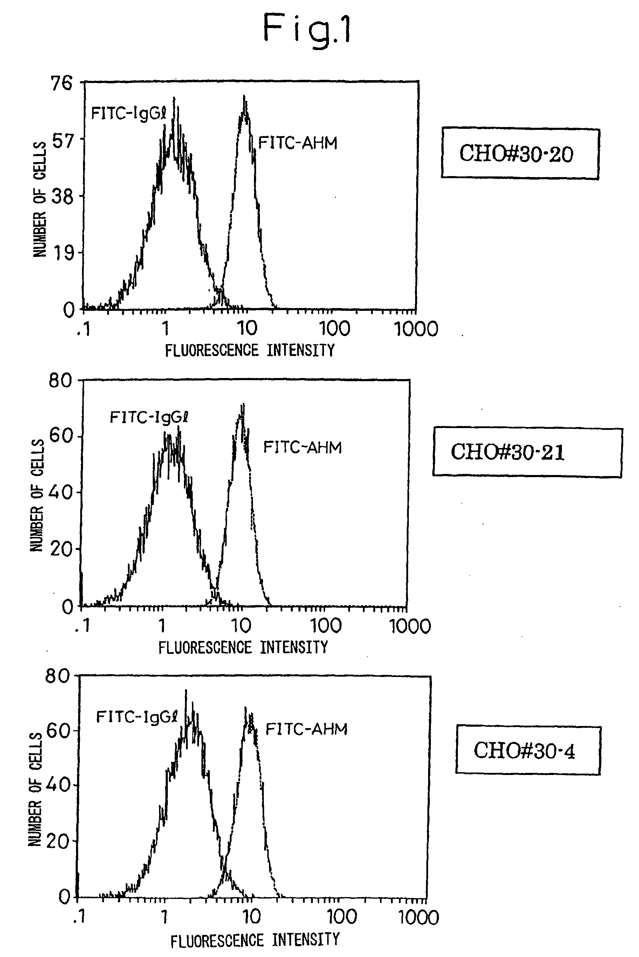

The gene introduction of pβ-gal-IRES2-EGFP-F is likely to change the properties of CHO #30 except for the expression of β-galactosidase and EGFP-F. Accordingly, it was confirmed whether or not the expression of the HM antigen for the CHO #30 cell lines was lost. Thus, after the cells of the cell line selected as above, and that stably express β-galactosidase, were scraped with typsin-EDTA, they were washed in PBS. They were then allowed to react with 4 μg of fluorescein-labeled IgG (FITC-IgG) or fluorescein-labeled anti-HM antibody (FITC-AHM) at 4°C for 1 hour and, after washing in PBS, they were analyzed by EPICS-XL-MCL with fluorescein (FITC) as an index. As a result, the expression of HM1.24 antigen was confirmed in CHO #30-20, CHO #30-21, and CHO #30-40 cell lines (Fig. 1).

(5) β-galactosidase activity and the amount of EGFP-F expressed of the cell line that stably expresses β-galactosidase

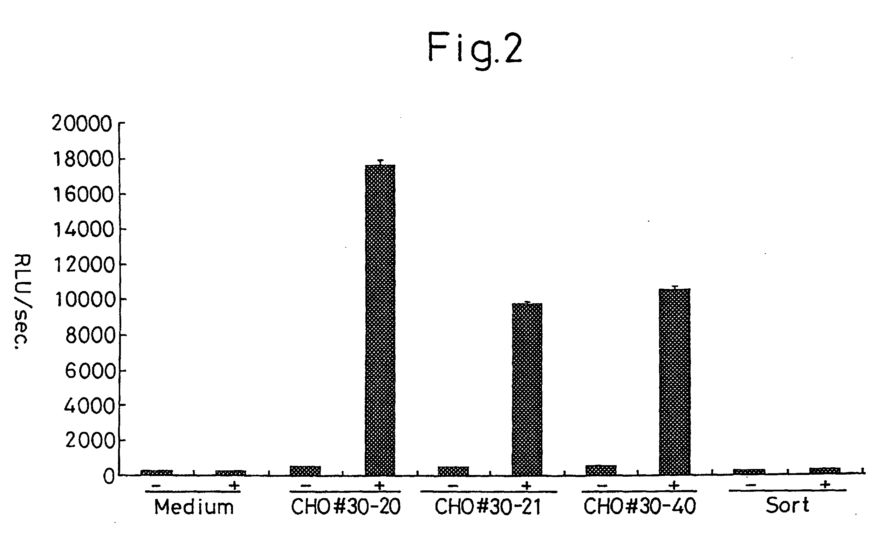

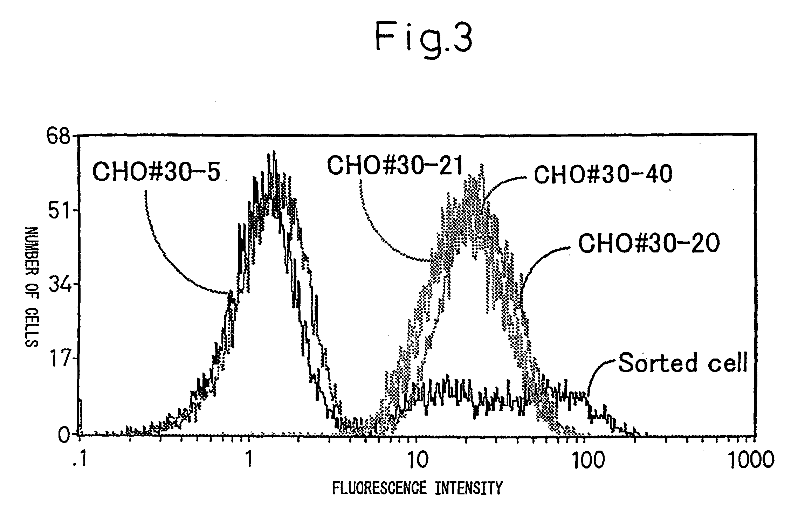

For the three cell lines obtained as the β-galactosidase high-expression line, the amount of β-galactosidase expressed and the amount of EGFP-F expressed were compared. As a result, the amount of β-galactosidase expressed and the amount of EGFP-F expressed were in the order of CHO #30-20 > CHO #30-40 > CHO #30-21, and a rank correlation was observed in the amount of β-galactosidase expressed and the amount of EGFP-F expressed (Fig. 2 and Fig. 3).

The amount of β-galactosidase expressed shown in Fig. 2 was determined for the cells measured as described in the above Fig. 2 for $1 \times 10^3$ cells in a manner similar to the one described in the above (2), and the amount of EGFP-F expressed shown in Fig. 3 was determined by detecting fluorescence at a wavelength of 525 nm using EPICS-XL-MCL.

(6) Expression stability of the CHO #30 line that highly expresses β-galactosidase

The amount of β-galactosidase expressed was compared when the CHO #30-20 cell line was cultured for a long time. As a result, subcultures after monocloning of the CHO #30 cell line stably expressed β-galactosidase for about two months (a mean of 16432.2 RLU/$1 \times 10^3$ cells, %CV 13.4).

Example 4. Method of determining ADCC activity using PBMC

As effector cells, monocytes, isolated from the peripheral blood of normal healthy donors by density centrifugation, were used. Thus, an equal amount of PBS was added to the peripheral blood of normal healthy donors, onto which Ficoll-Paque PLUS (Pharmacia) was layered, which was then centrifuged at 500 g for 30 minutes. After harvesting the monocyte phase and then washing three times with RPMI1640 containing 10% FBS (GIBCO), the cell number was adjusted to $5 \times 10^6$/ml with α-MEM medium containing 10% FCS.

After scraping with typsin-EDTA, 50 μl of $2 \times 10^5$ cells/ml of CHO #30 cell line that stably expresses β-galactosidase suspended in α-MEM containing 10% FCS and 50 μl of various concentrations of anti-HM1.24 antibody were added to a U-bottomed 96-well plate, and reacted at 4°C for 15 minutes. Then 100 μl of effector cells were added and cultured at 37°C for 4 hours. After culturing, 20 μl of the culture supernatant was collected, and β-galactosidase activity therein was determined. The maximum amount of enzyme released was set as the enzyme amount released from the cell lysis buffer of the Galacton-star assay kit.

Cytotoxicity was calculated as:

$$\text{Cytotoxicity}(\%)\ (\%\ \beta\text{-galactosidase}) = (A-C) \times 100 / (B-C)$$

wherein A represents a β-galactosidase activity (RLU/sec) released in the presence of antibody, B represents a β-galactosidase activity (RLU/sec) released from the cell lysis buffer, and C represents a β-galactosidase activity (RLU/sec) released only from the culture liquid containing no antibody.

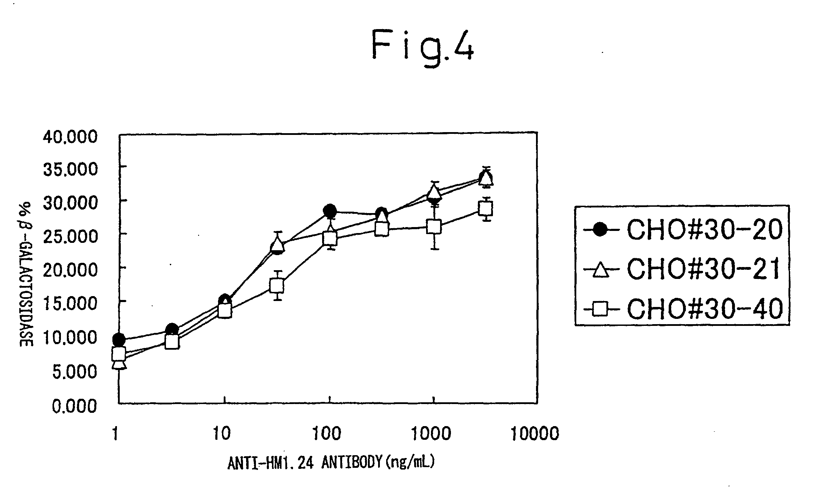

The result is shown in Fig. 4. It was confirmed that, in any of the cell lines that stably express β-galactosidase, β-galactosidase is released in a manner dependent on the concentration of anti-HM1.24 antibody, and cell death takes place in an antibody concentration-dependent manner.

Example 5. Development of activity based on the dissolution of a cell line that highly expresses β-galactosidase

The CHO #30 line that highly expresses β-galactosidase was plated to a density of 100, 625, 1000, 1250, 2500, 10000, and 20000/100 μl/well in eight wells each. To four wells of them, the lysis solution attached to the β-galactosidase detection kit (PE Biosystems, Cat. No. BM300S) was added, and to the remaining four wells, PBS was added at 100 μl/well, which were then cultured at 37°C for 1.5 hour in the presence of 5% $CO_2$. Then, 20 μl of the supernatant was added to 100 μl/well of the substrate of the β-galactosidase activity detection kit that had previously been dispensed, and then cultured for 1 hour at room temperature, and chemiluminescence was determined using ALVO-sx5. As a result, β-galactosidase activity was detected dependent on the cell number plated for the cells to which the lysis solution was added, whereas no activity was detected for those to which PBS was added.

Example 6. Effect of the E/T ratio on cytotoxicity of commercially available peripheral blood monocyte fraction stimulated with IL-2

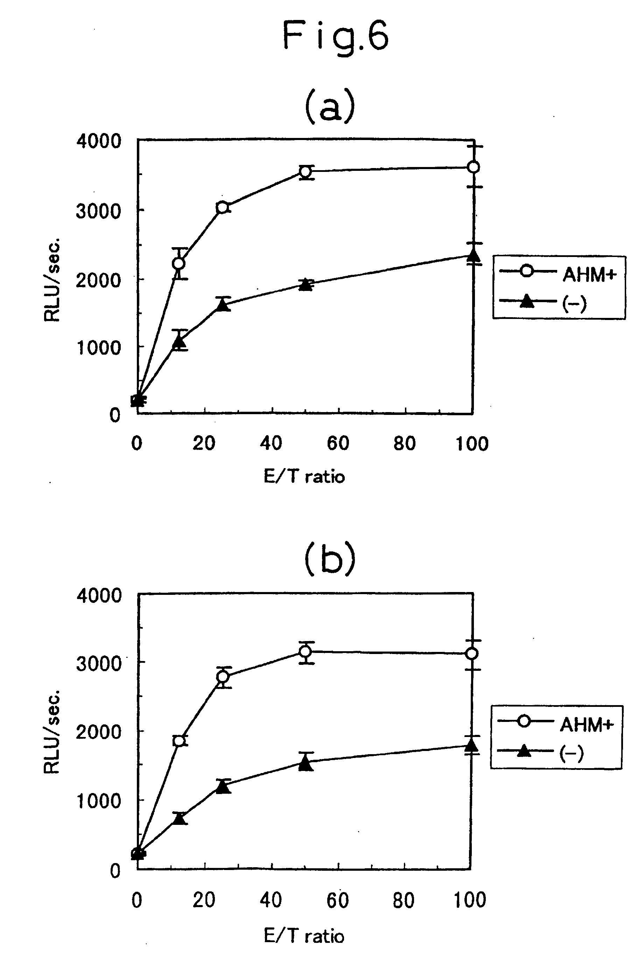

The CHO #30 line that highly expresses β-galactosidase was plated to a density of 5000/50 μl/well, to which 50 μl/well of α-MEM medium (nucleic acid added) containing anti-HM1.24 antibody (a final concentration of 1 or 0 μg/ml) was added, and the commercially available human peripheral blood monocyte fraction (BioWhittaker, Cat. NO. CC-2702) that had previously been cultured for 18 hours with 150 units/ml of IL-2 was added to 0, 6250, 12500, 25000, or 50000 cells/100 μl/well (effector/target ratio: 0, 12.5, 25, 50, 100), and cultured at 37°C for 4 hours in the presence of 5% $CO_2$. Then, 20 μl of the supernatant was added to 100 μl/well of the substrate of the β-galactosidase activity detection kit that had previously been dispensed, and then cultured for 1 hour at room temperature and chemiluminescence was determined using ALVO-sx5. The result is shown in Fig. 6. Even when no anti-HM1.24 antibody was added, β-galactosidase activity increased depending on the effector/target ratio (E/T ratio), and when anti-HM1.24 antibody was added, it increased by nearly two-fold. The difference between the two is equivalent to ADCC activity.

**Claims**

1. A method of determining cell death, of target cells, comprising incubating target cells that contain a gene encoding a membrane-bound protein capable of inducing cell death and a gene encoding a marker protein, and determining the marker protein that leaks out of the cells due to cell death.

2. The method according to claim 1 wherein said incubation is carried out in the presence of an antibody or a ligand against the above membrane-bound protein capable of inducing cell death.

3. The method according to claim 1 or 2 wherein said incubation is carried out in the presence of effector cells.

4. The method according to any one of the claims 1-3 wherein said target cells are cells that can provide a stable expression system of membrane-bound proteins.

5. The method according to claim 4 wherein said target cells are CHO cells.

6. The method according to any one of the claims 1-5 wherein said marker is an enzyme.

7. The method according to claim 6 wherein said marker is β-galactosidase.

8. A method of screening substances that either induce or inhibit cell death of target cells, comprising incubating target cells that contain a gene encoding a membrane-bound protein capable of inducing cell death and a gene encoding a marker protein together with test substances, and determining the marker protein that leaks out of the cells due to cell death.

9. The method according to claim 8 wherein said incubation is carried out in the presence of an antibody or a ligand against the above membrane-bound protein capable of inducing cell death.

10. The method according to claim 8 or 9 wherein said incubation is carried out in the presence of effector cells.

11. The method according to any one of the claims 8-10 wherein said target cells are cells that can provide a stable expression system of membrane-bound proteins.

12. The method according to claim 11 wherein said target cells are CHO cells.

13. The method according to any one of the claims 8-12 wherein said marker is an enzyme.

14. The method according to claim 13 wherein said marker is β-galactosidase.

15. Target cells for use in a method of determining cell death of target cells or screening substances that either induce or inhibit cell death of target cells, said cells containing a gene encoding a membrane-bound protein capable of inducing cell death and a gene encoding a marker protein, and leaking out of the cells due to cell death induced by reactions derived from said membrane-bound protein.

16. The method according to claim 8 wherein said incubation is carried out in the presence of an antibody or a ligand against the above membrane-bound protein capable of inducing cell death.

17. The method according to claim 15 or 16 wherein said incubation is carried out in the presence of effector cells.

18. The method according to any one of the claims 15-17 wherein said target cells are cells that can provide a stable expression system of membrane-bound proteins.

19. The method according to claim 18 wherein said target cells are CHO cells.

20. The method according to any one of the claims 15-19 wherein said marker is an enzyme.

21. The method according to claim 20 wherein said marker is β-galactosidase.

# Fig.1

# Fig.2

EP 1 344 831 A1

Fig.3

# Fig.4

# Fig.5

## (a)

## (b)

# Fig.6

## (a)

## (b)

<table>
<tr><td colspan="2" align="center"><b>INTERNATIONAL SEARCH REPORT</b></td><td>International application No.<br><br>PCT/JP01/11301</td></tr>
</table>

A. CLASSIFICATION OF SUBJECT MATTER

Int.Cl⁷ C12Q1/02, C12N15/09, C12N5/10, G01N33/15, G01N33/48,
G01N33/50, G01N33/566, G01N33/68

According to International Patent Classification (IPC) or to both national classification and IPC

B. FIELDS SEARCHED

Minimum documentation searched (classification system followed by classification symbols)

Int.Cl⁷ C12Q1/02, C12N15/09, C12N5/10, G01N33/15, G01N33/48,
G01N33/50, G01N33/566, G01N33/68

Documentation searched other than minimum documentation to the extent that such documents are included in the fields searched

Electronic data base consulted during the international search (name of data base and, where practicable, search terms used)

WPI(DIALOG), BIOSIS(DIALOG), MEDLINE(STN)

C. DOCUMENTS CONSIDERED TO BE RELEVANT

| Category* | Citation of document, with indication, where appropriate, of the relevant passages | Relevant to claim No. |
|---|---|---|
| Y | EP, 844483, A1 (Pasteur Merieux Serums & Vaccins SA), 27 May, 1998 (27.05.98), & FR 2755977 A1 & CA 2219725 A & JP 10-215868 A | 1-21 |
| Y | BACHY, M. et al., Beta galactosidase release as an alternative to chromium release in cytotoxic T-cell assays. J.Immunol.Methods. 1999, Vol.230, No.1-2, pages 37 to 46 | 1-21 |
| Y | OHTOMO, T. et al., Molecular cloning and characterization of a surface antigen preferentially overexpressed on multiple myeloma cells. Biochem. Biophys.Res.Commun. 1999, Vol.258, No.3, pages 583 to 591 | 1-21 |

☐ Further documents are listed in the continuation of Box C.  ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 April, 2002 (04.04.02) | 16 April, 2002 (16.04.02) |

| Name and mailing address of the ISA/<br>　Japanese Patent Office | Authorized officer |
|---|---|
| Facsimile No. | Telephone No. |

Form PCT/ISA/210 (second sheet) (July 1998)